# EUROPEAN PATENT SPECIFICATION

(11) **EP 1 192 137 B1**
(45) Date of publication and mention of the grant of the patent: **23.10.2013**
(21) Application number: 00946961.0
(22) Date of filing: 28.06.2000
(51) Int. Cl.: C07D 213/65, C07D 215/36, C07D 215/20, C07D 277/74, C07D 211/82, C07D 235/28, C07C 311/21, A61K 31/18, A61K 31/44, A61K 31/415, A61K 31/425

(54) **COMPOUNDS FOR THE MODULATION OF PPAR-GAMMA ACTIVITY**
VERBINDUNGEN ZUM MODULIEREN DER PPAR-GAMMA AKTIVITÄT
COMPOSES UTILISES POUR LA MODULATION DE L'ACTIVITE DE PPAR-GAMMA

(30) Priority: 30.06.1999 US 141672 P
(43) Date of publication of application: 03.04.2002
(73) Proprietor: Amgen Inc., Thousand Oaks, CA 91320-1799 (US); Japan Tobacco Inc., Takatsuki Osaka, Osaka 569-1125 (JP)
(72) Inventor: MCGEE, Lawrence, R., Pacifica, CA 94044 (US); HOUZE, Jonathan, B., San Mateo, CA 94402 (US); RUBENSTEIN, Steven, M., Pacifica, CA 94044 (US); HAGIWARA, Atsushi, Takatsuki Osaka 569-1125 (JP); FURUKAWA, Noboru, Takatsuki Osaka 569-1125 (JP); SHINKAI, Hisashi, Takatsuki Osaka 569-1125 (JP)
(74) Representative: Walker, Ross Thomson
(86) International application number: PCT/US2000/018178
(87) International publication number: WO 2001/000579

(56) References cited:
- EP-A- 0 069 585
- EP-A- 0 261 539
- EP-A- 0 749 751
- EP-A- 0 855 391
- WO-A-95/01326
- WO-A-96/15118
- WO-A-97/31907
- WO-A-99/06378
- WO-A-99/10320
- WO-A-99/38845
- LEHMANN J M ET AL: "PEROXISOME PROLIFERATOR-ACTIVATED RECEPTORS ALPHA AND GAMMA ARE ACTIVATED BY INDOMETHACIN AND OTHER NON-STEROIDAL ANTI-INFLAMMATORYDRUGS" JOURNAL OF BIOLOGICAL CHEMISTRY,US,AMERICAN SOCIETY OF BIOLOGICAL CHEMISTS, BALTIMORE, MD, vol. 272, no. 6, 7 February 1997 (1997-02-07), pages 3406-3410, XP002923816 ISSN: 0021-9258
- WILLSON T M ET AL: "THE STRUCTURE-ACTIVITY RELATIONSHIP BETWEEN PEROXISOME PROLIFERATOR-ACTIVATED RECEPTOR GAMMA AGONISM AND THE ANTIHYPERGLYCEMIC ACTIVITY OF THIAZOLIDINEDIONES" JOURNAL OF MEDICINAL CHEMISTRY,US,AMERICAN CHEMICAL SOCIETY. WASHINGTON, vol. 39, no. 3, 2 February 1996 (1996-02-02), pages 665-668, XP000613613 ISSN: 0022-2623
- FORMAN B M ET AL: "15-DEOXY-DELTA 12,14-PROSTAGLANDIN J2 IS A LIGAND FOR THE ADIPOXYTE DETERMINATION FACTOR PPARY" CELL,US,CELL PRESS, CAMBRIDGE, NA, vol. 83, 1 December 1995 (1995-12-01), pages 803-812, XP000575803 ISSN: 0092-8674
- DATABASE CHEMABS [Online] CAS; BURMISTROV K.S. ET AL.: retrieved from STN Database accession no. 122:132338 XP002148690 & ZH. ORG. KHIM., vol. 30, no. 5, 1994, pages 744-747,
- DATABASE CHEMABS [Online] CAS; SEBE, ION ET AL.: retrieved from STN Database accession no. 117:214517 XP002148691 & REV. CHIM., vol. 43, no. 5-6, 1992, pages 222-225,
- DATABASE CHEMABS [Online] CAS; BURMISTROV K.S. ET AL.: retrieved from STN Database accession no. 115:8165 XP002148692 & ZH. ORG. KHIM., vol. 26, no. 9, 1990, pages 1995-1998,
- DATABASE CHEMABS [Online] CAS; PIEPER H. ET AL.: retrieved from STN Database accession no. 112:138679 XP002148693 & ARZNEIM.-FORSCH., vol. 39, no. 9, 1989, pages 1073-1080,
- DATABASE CHEMABS [Online] CAS; BAGULEY BRUCE C. ET AL.: retrieved from STN Database accession no. 108:179602 XP002148694 & EUR. J. CANCER CLIN. ONCOL., vol. 24, no. 2, 1988, pages 205-210,
- DATABASE CHEMABS [Online] CAS; SARUL E. ET AL.: retrieved from STN Database accession no. 103:123106 XP002148695 & LATV. PSR ZINAT. AKAD. VESTIS, KIM. SER., vol. 2, 1985, pages 225-228,
- DATABASE CHEMABS [Online] CAS; WOLLWEBER H. ET AL.: retrieved from STN Database accession no. 101:151540 XP002148696 & ARZNEIM.-FORSCH., vol. 34, no. 5, 1984, pages 531-542,
- DATABASE CHEMABS [Online] CAS; DENNY WILLIAM A. ET AL.: retrieved from STN Database accession no. 96:79437 XP002148697 & J. MED. CHEM., vol. 25, no. 3, 1982, pages 276-315,
- DATABASE CHEMABS [Online] CAS; MYSYK D.D. ET AL.: retrieved from STN Database accession no. 92:163637 XP002148698 & ZH. ORG. KHIM., vol. 15, no. 12, 1979, pages 2499-2502,
- DATABASE CHEMABS [Online] CAS; ZAITSEVA ET AL.: retrieved from STN Database accession no. 86:43377 XP002148699 & ZH. ORG. KHIM., vol. 12, no. 9, 1976, pages 1987-1992,

## Description

### FIELD OF THE INVENTION

The present invention relates to compounds that modulate the PPARγ receptor and are useful in the diagnosis and treatment of type II diabetes (and complications thereof), hypercholesterolemia (and related disorders associated with abnormally high or low plasma lipoprotein or triglyceride levels) and inflammatory disorders.

### BACKGROUND OF THE INVENTION

The peroxisome proliferator-activated receptors (PPARs) are transducer proteins belonging to the steroid/thyroid/retinoid receptor superfamily. The PPARs were originally identified as orphan receptors, without known ligands, but were named for their ability to mediate the plelotropic effects of fatty acid peroxisome proliferators. These receptors function as ligand-regulated transcription factors that control the expression of target genes by binding to their responsive DNA sequence as heterodimers with RXR. The target genes encode enzymes involved in lipid metabolism and differentiation of adipocytes. Accordingly, the discovery of transcription factors involved in controlling lipid metabolism has provided insight into regulation of energy homeostasis in vertebrates, and further provided targets for the development of therapeutic agents for disorders such as obesity, diabetes and dyslipidemia.

PPARγ is one member of the nuclear receptor superfamily of ligand-activated transcription factors and has been shown to be expressed in an adipose tissue-specific manner, Its expression is induced early during the course of differentiation of several preadipocyte cell lines. Additional research has now demonstrated that PPARγ plays a pivotal role in the adipogenic signaling cascade. PPARγ also regulates the ob/leptin gene which is involved in regulating energy homeostasis, and adipocyte differentiation which has been shown to be a critical step to be targeted for anti-obesity and diabetic conditions.

In an effort to understand the role of PPARγ in adipocyte differentiation, several investigators have focused on the identification of PPARγ activators. One class of compounds, the thiazolidinediones, which were known to have adipogenic effects on preadipocyte and mesenchymal stem cells *in vitro,* and antidiabetic effects in animal models of non-insulin-dependent diabetes mellitus (NIDDM) were also demonstrated to be PPARγ-selective ligands. More recently, compounds that selectively activate murine PPARγ were shown to possess *in vivo* antidiabetic activity in mice.

Despite the advances made with the thiazolidinedione class of antidiabetes agents, unacceptable side effects have limited their clinical use. Accordingly, there remains a need for potent, selective activators of PPARγ which will be useful for the treatment of NIDDM and other disorders related to lipid metabolism and energy homeostasis. Still further, compounds that block PPARγ activity would be useful for interfering with the maturation of preadipocytes into adipocytes and thus would be useful for the treatment of obesity and related disorders associated with undesirable adipocyte maturation. Surprisingly, the present invention provides compounds that are useful as activators as well as antagonists of PPARγ activity and compositions containing them, along with methods for their use.

### DETAILED DESCRIPTION OF THE INVENTION

### Abbreviations and Definitions:

The following abbreviations are used herein: PPARγ: peroxisome proliferator-activated receptor γ; NIDDM: non-insulin-depondent diabetes mellitus; Et₃N: triethylamine; MeGH: methanol; and DMSO: dimethylsulfoxide.

The term "alkyl," by itself or as part of another substituent, means, unless otherwise stated, a straight or branched chain, or cyclic hydrocarbon radical, or combination thereof, which may be fully saturated, mono- or polyunsaturated and can include di- and multivalent radicals, having the number of carbon atoms designated *(i.e.* C₁-C₁₀ means one to ten carbons). Examples of saturated hydrocarbon radicals include groups such as methyl, ethyl, n-propyl, isopropyl, n-butyl, t-butyl, isobutyl, sec-butyl, cyclohexyl, (cyclohexyl)ethyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below as "heteroalkyl," "cycloalkyl" and "alkylene." The term "alkylene" by itself or as part of another substituent means a divalent radical derived from an alkane, as exemplified by -CH₂CH₂CH₂CH₂-. Typically, an alkyl group will have from t to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl," by itself or in combination with another term, means, unless otherwise stated, a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms are optionally oxidized and the nitrogen heteroatom may optionally be quaternized. The heteroatom(s) O, N and S may be placed at any interior position of the heteroalkyl group. The heteroatom Si may be placed at any position of the heteroalkyl group, including the position at which the alkyl group is attached to the remainder of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. Up to two heteroatoms may be consecutive, such as, for example, -CH₂-NH-OCH₃ and -CH₂-O-Si(CH₃)₃. Also included in the term "heteroalkyl" are those radicals described in more detail below as "heteroalkylene" and "heterocycloalkyl." The term "heteroalkylene" by itself or as part of another substituent means a divalent radical derived from heteroalkyl, as exemplified by -CH₂-CH₂-S-CH₂CH₂- and -CH₂-S-CH₂-CH₂-NH-CH₂-. For heteroalkylene groups, heteroatoms can also occupy either or both of the chain termini. Still further, for alkylene and heteroalkylene linking groups, as well as all other linking group provided in the present invention, no orientation of the linking group is implied.

The terms "cycloalkyl" and "heterocycloalkyl", by themselves or in combination with other terms, represent, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include cyclopentyl, cyclohexyl, 1-cyclohexenyl, 3-cyclohexenyl, cycloheptyl, and the like. Examples of heterocycloalkyl include 1 - (1,2,5,6-tetrahydropyridyl), 1 -piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "fluoroalkyl," are meant to include monofluoroalkyl and polyfluoroalkyl.

The term "aryl," employed alone or in combination with other terms (e.g., aryloxy, arylthioxy, arylalkyl) means, unless otherwise stated, an aromatic substituent which can be a single ring or multiple rings (up to three rings) which are fused together or linked covalently. The rings may each contain from zero to four heteroatoms selected from N, O, and 5, wherein the nitrogen and sulfur atoms are optionally oxidized, and the nitrogen atom(s) are optionally quaternized. The aryl groups that contain heteroatoms may be referred to as "heteroaryl" and can be attached to the remainder of the molecule through a heteroatom Non-limiting examples of aryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 2-benzothiazolyl, 5-benzothiazolyl, 2-benzoxazolyl, 5-benzoxazolyl, purinyl, 2-benzimidazolyl, 5-indolyl, 1-isoquinolinyl, 5-isoquinolinyl, 2-quinoxalinyl, 5-quinoxalinyl, 3-quinolinyl, and 6-quinolinyl. Substituents for each of the above noted aryl ring systems are selected from the group of acceptable substituents described below. The term "arylalkyl" is meant to include those radicals in which an aryl group is attached to an alkyl group (e.g., benzyl, phenethyl, pyridylmethyl and the like) or a heteroalkyl group (*e.g*., phenoxymethyl, 2-pyridyloxymethyl, 3-(1-naphthyloxy)propyl, and the like).

Each of the above terms *(e.g.,* "alkyl," "heteroalkyl" and "aryl") are meant to include both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be a variety of groups selected from: -OR', =O, =NR', =N-OR', -NR'R", -SR', -halogen, -SiR'R"R"',-OC(O)R', -C(O)R', -CO₂R', CONR'R", -OC(O)NR'R", -NR"C(O)R', -NR'-C(O)NR"R"', -NR"C(O)₂R', -NH-C(NH₂)=NH, -NR'C(NH₂)=NH, -NH-C(NH₂)=NR',-S(O)R', -S(O)₂R', -S(O)₂NR'R", -CN and -NO₂ in a number ranging from zero to (2N+ 1), where N is the total number of carbon atoms in such radical. R', R" and R"' each independently refer to hydrogen, unsubstituted(C₁-C₈)alkyl and heteroalkyl, unsubstituted aryl, aryl substituted with 1-3 halogens, unsubstituted alkyl, alkoxy or thioalkoxy groups, or aryl-(C₁-C₄)alkyl groups. When R' and R" are attached to the same nitrogen atom, they can be combined with the nitrogen atom to form a 5-, 6-, or 7-membered ring. For example, -NR'R" is meant to include 1-pyrrolidinyl and 4-morpholinyl. From the above discussion of substituents, one of skill in the art will understand that the term "alkyl" is meant to include groups such as haloalkyl (e.g., -CF₃ and -CH₂CF₃) and acyl (e.g.,-C(O)CH₃, -C(O)CF₃, -C(O)CH₂OCH₃, and the like). Preferably, the alkyl groups (and related alkoxy, heteroalkyl, etc.) are unsubstituted or have 1 to 3 substituents selected from halogen, -OR', =O, -NR'R", -SR', -OC(O)R', -C(O)R', -CO₂R', -CONR'R", -NR"C(O)R', -S(O)₂R', -S(O)₂NR'R", -CN and -NO₂. More preferably, the alkyl and related groups have 0, 1 or 2 substituents selected from halogen, -OR', =O, -NR'R", -SR', -CO₂R', -CONR'R", -NR"C(O)R', -CN and -NO₂.

Similarly, substituents for the aryl groups are varied and are selected from halogen, -OR', -OC(O)R', -NR'R", -SR', -R', -CN, -NO₂- -CO₂R', -CONR'R", -C(O)R', -OC(O)NR'R", -NR"C(O)R', -NR"C(O)₂R', -NR'-C(O)NR"R"', -NH-C(NH₂)=NH, -NR'C(NH₂)=NH, -NH-C(NH₂)=NR', -S(O)R', -S(O)₂R', -S(O)₂NR'R", -N₃, -CH(Ph)₂, perfluoro(C₁-C₄)alkoxy, and perfluoro(C₁-C₄)alkyl, in a number ranging from zero to the total number of open valences on the aromatic ring system; and where R', R" and R'" are independently selected from hydrogen, (C₁-C₈)alkyl and heteroalkyl, unsubstituted aryl, (unsubstituted aryl)-(C₁-C₄)alkyl, and (unsubstituted aryl)oxy-(C₁-C₄)alkyl. Preferably, the aryl groups are unsubstituted or have from 1 to 3 substituents selected from halogen,-OR', -OC(O)R', -NR'R", -SR', -R', -CN, -NO₂- -CO₂R', -CONR'R", -C(O)R',-NR"C(O)R', -S(O)₂R', -S(O)₂NR'R", perfluoro(C₁-C₄)alkoxy, and perfluoro(C₁-C₄)alkyl. Still more preferably, the aryl groups have 0, 1 or 2 substituents selected from halogen, -OR', -NR'R", -SR', -R', -CN, -NO₂- -CO₂R', -CONR'R", -NR"C(O)R', -S(O)₂R', -S(O)₂NR'R", perfluoro(C₁-C₄)alkoxy, and perfluoro(C₁-C₄)alkyl.

Two of the substituents on adjacent atoms of the aryl ring may optionally be replaced with a substituent of the formula wherein T and U are independently -NH-, - O**-,** -CH₂- or a single bond, and q is an integer of from 0 to 2. Alternatively, two of the substituents on adjacent atoms of the aryl ring may optionally be replaced with a substituent of the formula -A-(CH₂)ᵣ-B-, wherein A and B are independently -CH₂-, -O-,-NH-, -S-, -5(O)-, -S(O)₂₋, -S(O)₂NR'- or a single bond, and r is an integer of from 1 to 3. One of the single bonds of the new ring so formed may optionally be replaced with a double bond. Alternatively, two of the substituents on adjacent atoms of the aryl ring may optionally be replaced with a substituent of the formula -(CH₂),-X-(CH₂)ₜ-, where s and t are independently integers of from 0 to 3, and X is -O-, -NR'-, -S-, -S(O)-, -S(O)₂-, or -S(O)₂NR'-. The substituent R' in -NR'- and -S(O)₂NR'- is selected from hydrogen or unsubstituted (C₁-C₆)alkyl.

As used herein, the term "heteroatom" is meant to include oxygen (O), nitrogen (N), sulfur (S) and silicon (Si).

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, oxalic, maleic, malonic, benzoic, succinic, suberic, fumaric, mandelic, phthalic, benzeriesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S.M., et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present disclosure provides compounds which are in a prodrug form Prodrugs of the compounds described herein are those compounds that readily undergo chemical changes under physiological conditions to provide the compounds of the present disclosure. Additionally, prodrugs can be converted to the compounds of the present disclosure by chemical or biochemical methods in an *ex vivo* environment. For example, prodrugs can be slowly converted to the compounds of the present disclosure when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are intended to be encompassed within the scope of the present invention Certain compounds of the present invention may exist in multiple crystalline or amorphous forms. In general, all physical forms are equivalent for the uses contemplated by the present invention and are intended to be within the scope of the present invention

Certain compounds of the present invention possess asymmetric carbon atoms (optical centers) or double bonds; the racemates, diastereomers, geometric isomers and individual isomers are all intended to be encompassed within the scope of the present invention

The compounds of the present invention may also contain unnatural proportions of atomic isotopes at one or more of the atoms that constitute such compounds For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

### General:

A new class of compounds that interact with PPARγ has now been discovered. Depending on the biological environment *(e.g*., cell type, pathological condition of the host, *etc*.), these compounds can activate or block the actions of PPARγ. By activating the PPARγ receptor, the compounds will find use as therapeutic agents capable of modulating conditions mediated by the PPARγ receptor As noted above, example of such conditions is NIDDM. Additionally, the compounds are useful for the prevention and treatment of complications of diabetes (*e.g.,* neuropathy, retinopathy, glomerulosclerosis, and cardiovascular disorders), and treating hyperlipidemia. Still further, the compounds are useful for the modulation of inflammatory conditions which most recently have been found to be controlled by PPARγ (see, Ricote, at al., Nature, 391:79-82 (1998) and Jiang, et al., Nature, 391:82-86 (1998). Examples of inflammatory conditions include rheumatoid arthritis and atherosclerosis.

Compounds that act via antagonism of PPARγ are useful for treating obesity, hypertension, hyperlipidemia, hypercholesterolemia, hyperlipoproteinemia, and metabolic disorders.

### Embodiments of the invention:

In one aspect, the present invention provides compounds which are represented by the formula: 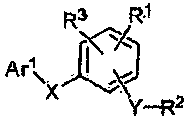 or a pharmaceutically acceptable salt thereof, wherein
Ar¹ is a substituted or unsubstituted 3-quinolinyl;
X is -O-, -NH-, or -S-;
Y is -NH-S(O)₂-;
R¹ is a member selected from the group consisting of halogen, (C₁-C₈)alkyl, (C₁-C₈)alkoxy, -C(O)R¹⁴, -CO₂R¹⁴, and -C(O)NR¹⁵R¹⁶, wherein
R¹⁴ is a member selected from the group consisting of hydrogen, (C₁-C₈)alkyl, (C₂-C₈)heteroalkyl; wherein heteroalkyl means a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms are optionally oxidized and the nitrogen atom may be optionally quartemized, the heteroatoms O, N and S may be placed at any interior position of the heteroalkyl group; aryl, and aryl(C₁-C₄)alkyl;
R¹⁵ and R¹⁶ are members independently selected from the group consisting of hydrogen, (C₁-C₈)alkyl, (C₂-C₈)heteroalkyl; wherein heteroalkyl means a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms are optionally oxidized and the nitrogen atom may be optionally quarternized, the heteroatoms O, N and S may be placed at any interior position of the heteroalkyl group; aryl, and aryl(C₁-C₄)alkyl, or taken together with the nitrogen to which each is attached form a 5-, 6-, or 7-membered ring;
R² is a phenyl group having from 0 to 3 substituents selected from the group consisting of halogen, -OCF₃, -OH, -O(C₁-C₈)alkyl, -C(O)-(C₁-C₈)alkyl, -CN, -CF₃, (C₁-C₈)alkyl, and -NH₂; and
R³ is a member selected from the group consisting of halogen, methoxy and trifluoromethoxy.

Preferably, represented by a formula selected from the group consisting of 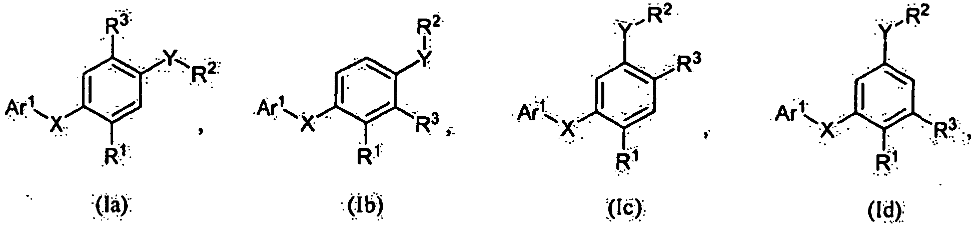 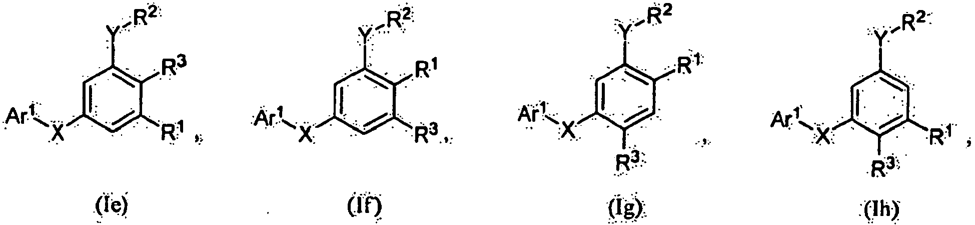 and

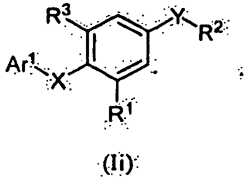

Further preferably, represented by the formula

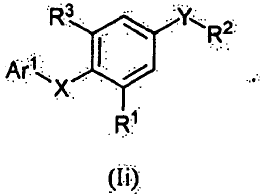

Advantageously, wherein R¹ is a member selected from the group consisting of halogen, (C₁-C₈)alkyl, and (C₁-C₈)alkoxy; R² is a phenyl group having from 0 to 3 substituents selected from the group consisting of halogen, -OCF₃, -OH, -O(C₁-C₈)alkyl, -C(O)-(C₁-C₈)alkyl, -CN, -CF₃, (C₁-C₈)alkyl, and -NH₂; and R³ is selected from the group consisting of halogen, methoxy and trifluoromethoxy.

Preferably, wherein R² is a phenyl group having from 0 to 3 substituents selected from the group consisting of halogen, -OCF₃, and -CF₃.

Further preferably, wherein R¹ and R³ are, each independently a halogen and R² is a phenyl group having from 0 to 3 substituents selected from the group consisting of halogen, -OCF₃, and -CF₃.

Advantageously, wherein the compound is of the formula

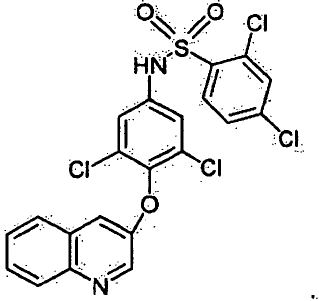

Preferably, wherein the quinolinyl is unsubstituted.

In another aspect of the present invention, there is provided a composition comprising a pharmaceutically acceptable excipient and a compound according to any one of the above embodiments.

In a further aspect of the present invention, there is provided the use of a compound of any one of the above embodiments for the manufacture of a medicament for use in modulating a metabolic disorder or inflammatory condition in a host

In a further aspect of the present invention; there is provided a compound of any one of the above embodiments for use in modulating a metabolic disorder or inflammatory condition in a host.

Preferably,-wherein said host is a mammal selected from the group, consisting of humans, dogs, monkeys, mice, rats, horses and cats.

Further preferably, wherein said compound is formulated for oral administration

Advantageously, wherein said compound is formulated for topical administration.

Preferably, wherein said modulating prevents a PPARγ-mediated condition.

Further preferably, wherein said disorder or condition is selected from the group consisting of NIDDM, obesity, and hypercholesterolemia and other lipid-mediated diseases, and inflammatory conditions.

Advantageously, wherein said compound is formulated for parenteral administration.

Preferably, wherein said metabolic disorder is mediated by PPARγ.

Further preferably, wherein said metabolic disorder is NIDDM.

Advantageously, wherein said inflammatory condition is rheumatoid arthritis or atherosclerosis.

Preferably, wherein the host is human

### Preparation of the Compounds

The compounds of the present invention can be prepared using standard synthetic methods. For exemplary purposes, Scheme 1 illustrates methods for the preparation of compounds of structural formula (Ia). One of skill in the art will understand that similar methods can be used for the synthesis of compounds in the other structural classes.

As shown in Scheme 1, compounds of the present invention can be prepared beginning with commercially available 2-chloro-5-nitrobenzonitrile (i). Treatment of J with a phenol, thiophenol, or optionally protected aniline in the presence of base and heat provides the adduct (ii). Reduction of the nitro group in ii with, for example, H₂ in the presence of Raney nickel catalyst provides an aniline derivative (iii). Sulfonylation of iii with an appropriate arylsulfonyl halide (Ar¹ 50₂C₁) in the presence of base (typically a tertiary amine) provides a target compound (iv). Compound iii can also be converted to a related compound of formula (vi) in which the orientation of the sulfonamide linkage is reversed. Thus, conversion of the aniline iii to the benzenesulfonyl chloride v can be accomplished using methods described in Hoffman, Organic Syntheses Collective Volume VII, p. 508-511. Subsequent treatment of v with an appropriate aniline provides the target compound vi.

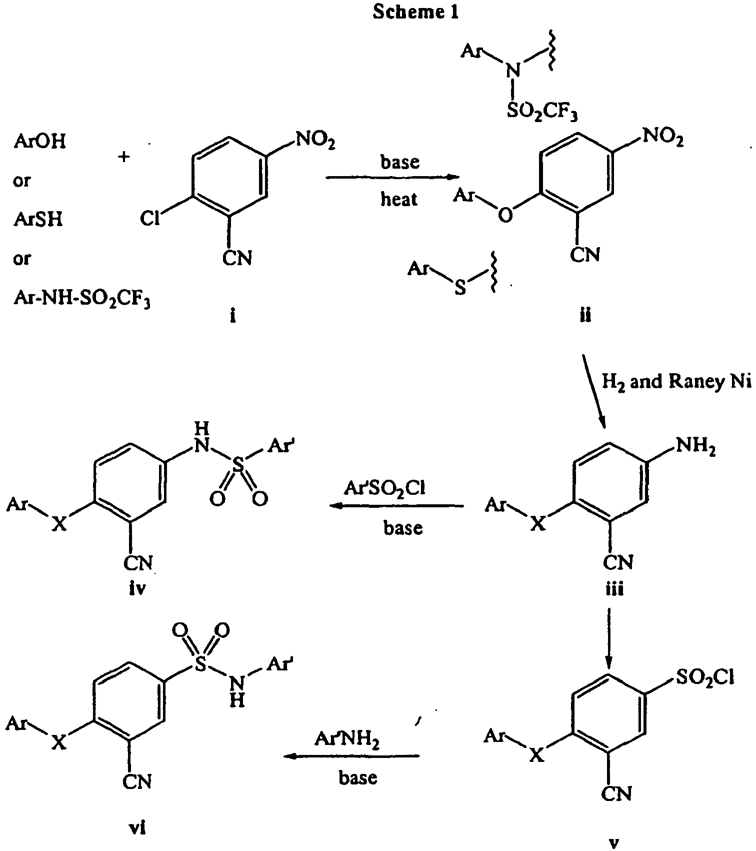

Other compounds of the present invention can be prepared beginning with, for example, 3,4-difluoronitrobenzene, 3-chloro-4-fluoronitrobenzene, 2-chloro-5-nitroanisole, 3-bromo-4-fluoronitrobenzene and the like.

### Analysis of the Compounds

The compounds of the present invention can be evaluated for modulation of the PPARγ receptor using assays such as those described in Jiang, et al., Nature 391:82-86 (1998), Ricote, et al., Nature 391:79-82 (1998) and Lehmann, et al., J. Bio/. Chem. 270(12): 12953-12956 (1995). Alternatively, the compounds can be evaluated for their ability to displace radiolabeled BRL 49653 from a PPARγ-GST fusion protein as follows:

### Materials:

PPARγ-GST fusion protein (prepared according to standard procedures), [³H]-BRL 49653 having 50 Ci/mmol specific activity, Polyfiltronics Unifilter 350 filtration plate and glutathione-Sepharose® beads (from Pharmacia: washed twice with 10x binding buffer in which BSA and DTI can be left out).

### Method:

Binding buffer (10 mM Tris-HCl, pH 8.0, 50 mM KCI, 10 mM DTT, 0.02% BSA and 0.0 1% NP-40) is added in 80 microliter amounts to the wells of the filtration plate. The test compound is then added in 10 microliters of DMSO. The PPARγ-GST fusion protein and radiolabeled BRL compound are premixed in binding buffer containing 10 mM DTT and added in 10 microliter amounts to the wells of the plate to provide final concentrations of 1 µg/well of PPARγ-GST fusion protein and 10 nM [³H]-BRL 49653 compound. The plate is incubated for 15 minutes. Glutathione-agarose bead is added in 50 µL of binding buffer, and the plate is vigorously shaken for one hour. The plate is washed four times with 200 µL/well of binding buffer (without BSA and DTT). The bottom of the plate is sealed and 200 µL/well of scintillation cocktail is added. The top of the plate is then sealed and the radioactivity is determined.

### Formulation and Administration of the Compounds (compositions)

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermally. Accordingly, the present invention also provides pharmaceutical compositions comprising a pharmaceutically acceptable carrier or excipient and either a compound of formula (I) or a pharmaceutically acceptable salt of a compound of formula (I).

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired.

The powders and tablets preferably contain from 5% or 10% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 1000 mg, preferably 1.0 mg to 100 mg according to the particular application and the potency of the active component. The composition can, if desired, also contain other compatible therapeutic agents.

In therapeutic use for the treatment of obesity, NIDDM, or inflammatory conditions, the compounds utilized in the pharmaceutical method of the invention are administered at the initial dosage of about 0.001 mg/kg to about 100 mg/kg daily. A daily dose range of about 0.1 mg/kg to about 10 mg/kg is preferred. The dosages, however, may be varied depending upon the requirements of the patient, the severity of the condition being treated, and the compound being employed. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. For convenience, the total daily dosage may be divided and administered in portions during the day, if desired.

The following examples are offered by way of illustration and are not intended to limit the scope of the invention.

### EXAMPLES

Reagents and solvents used below can be obtained from commercial sources such as Aldrich Chemical Co. (Milwaukee, Wisconsin, USA). ¹H-NMR spectra were recorded on a Varian Gemini 400 MHz NMR spectrometer. Significant peaks are tabulated in the order: number of protons, multiplicity (s, singlet; d, doublet; t, triplet; q, quartet; m, multiplet; br s, broad singlet) and coupling constant(s) in Hertz. Electron Ionization (EI) mass spectra were recorded on a Hewlett Packard 5989A mass spectrometer. Mass spectrometry results are reported as the ratio of mass over charge, followed by the relative abundance of each ion (in parentheses). In tables, a single m/e value is reported for the M+H (or as noted M-H) ion containing the most common atomic isotopes. Isotope patterns correspond to the expected formula in all cases. Electrospray ionization (ESI) mass spectrometry analysis was conducted on a Hewlett-Packard 1100 MSD electrospray mass spectrometer using the HP1 100 HPLC for sample delivery. Normally the analyte was dissolved in methanol at 0.1mg/mL and 1 microliter was infused with the delivery solvent into the mass spectrometer which scanned from 100 to 1500 daltons. All compounds could be analyzed in the positive ESI mode, using 1:1 acetonitrile/water with 1% acetic acid as the delivery solvent. The compounds provided below could also be analyzed in the negative ESI mode, using 2mM NH₄OAc in acetonitrile/water as delivery solvent.

**Abbreviations:** N-hydroxybenzotriazole (HOBT), 2-(1H-benzotriazole-1-yl)- 1,1,3,3-tetramethyluronium hexafluorophosphate (HBTU), N-methylmorpholine (NMM),:1-hydroxy-7-azabenzotriazole (HOAT), O-(7-azabenzotriazole-1-yl)-N,N,N' ,N'--tetramethyluronium hexafluorophosphate (HATU), 1-(3 -dimethylaminopropyl)-3-ethylcarbodiimide hydrochloride (EDCI).

### EXAMPLE 85

This illustrates the synthesis of compound **85.3**

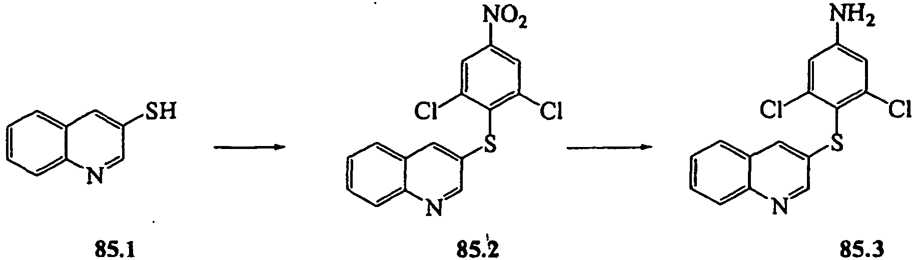

Compound **85.1** was prepared by a modification of the published procedure of Albert and Barlin (J. Chem. Soc. 2384-2396 (1959). 3-Aminoquinoline (15.0 g, 105 mmol) was suspended in a mixture of 10N HCl (40 mL), ice (21g) and water (100 mL) at 0-5 °C, before sodium nitrite (7.6 g, 110 mmol) was added slowly. The mixture was then added portionwise to another solution of potassium ethyl xanthate (20.8 g, 125 mmol) in water (60 mL) at 45 °C. The mixture was heated for 1 hr before cooling off. The mixture was then extracted with ether. The ethereal solution was washed with 2N NaOH solution, water, and brine before drying over magnesium sulfate. After filtration, the removal of the solvent gave a brown oil (15g), which was then dissolved in ethanol (150 mL) and refluxed with KOH (25g) under nitrogen overnight. The ethanol solvent was then removed under vacuum, and the residue was separated between water and ether. The ethereal solution was discarded. The aqueous solution was acidified to pH = ~4, before it was extracted with ether. Then ethereal solution was washed with brine, dried over magnesium sulfate, filtered and concentrated under vacuum to give crude product (7.5g) as a brown oil. Subsequent flash chromatography with eluent (0%-5%-10% ethyl acetate / dichloromethane) produced 3-mercaptoquinoline (85.1) (5.35g, 32% yield) as a solid.
¹H NMR (DMSO) δ 9.02 (1H, d, *J =* 2.3 Hz), 8.63 (1H, d, *J* = 2.2 Hz), 7.95-8.05 (2H, m), 7.75-8.02 (1H, m), 7.60-7.67 (1H, m).

To a mixture of 3-mercaptoquinoline (85.1)(1.18 g, 7.33 mmol) and 1,2,3-chloro-5-nitrobenzene (1.66 g, 7.33 mmol) dissolved in ethanol (100 mL), was added a THF solution of t-BuOK (7.5 mL, 1M). The mixture was then heated at 80 °C overnight before cooling off. After the removal of ethanol solvent, the mixture was separated between ethyl acetate and water. The organic solution was washed with brine, dried over magnesium sulfate and filtered. The filtrate was then concentrated to give a crude product, which was then flash chromatographed with eluent (10% hexanes / dichloromethane) to afford 85.2 (1.80 g, 70% yield) as a yellow oil.
¹H NMR (DMSO) δ 8.75 (1H, d, *J* = 2.3), 8.51 (1H, s), 8.22 (1H, s), 8.01 (1H, d, *J* = 8.4 Hz), 7.92 (1H, d, *J* = 7.6 Hz), 7.74-7.80 (1H, m), 7.60-7.66 (1H, m).

An ethyl acetate solution (100 mL) of 85.2 (1.80 g, 5.1 mmol) and tin chloride (II) dihydrate (6.88 g, 30 mmol) was heated at reflux overnight before cooling off. The solution was then poured into 1N NaOH solution (400 mL). After stirring for 30 min, the mixture was separated, and the organic solution was washed with water, saturated sodium bicarbonate and brine. After drying over magnesium sulfate, the solution was filtered and concentrated under vacuum. The residue was mixed with dichloromethane (10 mL) and sonicated. Subsequent vacuum filtration provided the aniline 85.3 (1.35g, 82% yield) as an off-white solid.
¹H NMR (DMSO) δ 8.61 (1H, d, *J =* 2.4), 7.96 (1H, d, *J=* 8.4 Hz), 7.88 (1H, d, *J =* 8.2 Hz), 7.83 (1H, d, *J =* 2.2 Hz), 7.67-7.72 (1H, m), 7.54-7.60 (1 H, m). mp 213.2 °C.

### EXAMPLE 86

This illustrates the synthesis of compound 86 (see Table 16).

The aniline **85.3** (250 mg, 0.78 mmol) and 2-chlorobenzenesufonyl chloride (339 mg, 1.60 mmol) were dissolved in a mixed solvent of THF (5 mL) and dichloromethane (5 mL). To the solution was added pyridine (0.185 mL, 2.34 mmol) and catalytic amount of DMAP. The solution was heated at 50 °C to distill off dichloromethane, and then THF with assistance of vacuum. The residue was flash chromatographed with eluent (2.5% ethyl acetate / dichloromethane) to give sulfonamide **86** (302 mg, 78%) as an off-white solid.
¹H NMR(DMSO) δ 11.58 (1H, s), 8.61 (1H, d, *J =* 2.4 Hz), 8.19 (1H, d, *J* = 7.6 Hz), 7.83-8.00 (3H, m), 7.67-7.75 (3H, m), 7.56-7.65 (2H, m), 7.31 (2H, s). MS (M+H) 494.9. mp: 219.6 °C. Anal. calcd: C 50.87, H 2.64, N 5.65; found C 50.86, H 2.62, N 5.52.

The compounds of Table 16 were prepared by the method of example **86** from compound **84.3** and the corresponding arylsulfonyl chloride.

**Table 16**

| | | | | | | |
|---|---|---|---|---|---|---|
| 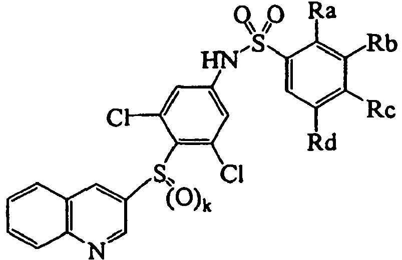 | | | | | | |

| | **k** | **Rₐ** | **R_{b}** | **R_{c}** | **R_{d}** | **m/e (M+H)** |
|---|---|---|---|---|---|---|
| **86** | 0 | Cl | H | H | H | 495 |
| **87.1** | 0 | Cl | H | Cl | H | 529 |
| **87.2** | 0 | H | H | H | H | 461 |
| **87.3** | 0 | Cl | H | CF₃ | H | 561 (M-H) |
| **88.1** | 1 | Cl | H | H | H | 511 |
| **88.2** | 1 | Cl | H | Cl | H | 543 (M-H) |
| **88.3** | 1 | H | H | H | H | 477 |

### EXAMPLE 87

### Example 87.1

¹H NMR(DMSO) δ 11.66 (1H, broad), 8.63 (1H, d, J= 2.3 Hz), 8.18 (1H, d, J= 8.6 Hz), 7.85-8.00 (4H, m), 7.70-7.75 (2H, m), 7.57-7.62 (1H, m), 7.32 (2H, s). MS (M+H) 529.0. mp 214.0 °C. Elemental Analysis: theory C 47.56, H 2.28, N 5.28; found C47.30, H 2.36, N 5.37.

### Example 87.2

¹H NMR(DMSO): δ 11.22 (1H, s), 8.61 (1H, d, *J* = 2.3 Hz), 7.82-7.98 (5H, m), 7.57-7.75 (5H, m), 7.34 (2H, s). MS (M+H) 461.0. mp 246.8 °C. Elemental Analysis theory C 54.67, H 3.06, N 6.07; found C 54.71, H 3.05, N 5.94.

### Example 87.3

¹H NMR (DMSO) δ 11.70-12.00 (1 H, broad), 8.60-8.67 (1H, m), 8.35-8.43 (1H, m), 8.20-8.25 (1H, m), 7.56-8.06 (6H, m), 7.32-7.38 (2H, m). MS (M-H) 560.9. mp: 225.1 °C. Elemental Analysis: theory C 46.86, H 2.15, N 4.97; found C. 47.01, H 2.26, N 4.98.

### EXAMPLE 88

### General procedure for sulfur oxidation to the sulfoxide:

A naphthylthioether of examples 86 or 87 (0.2 mmol) was dissolved in a mixed solvent of dichloromethane (10 mL) and methanol (5 mL). To the solution was added mCPBA (120 mg, 0.7 mmol, 77% pure) in six batches over 20 minute intervals. Then the solution was washed with 5% sodium thiosulfate solution, 1% sodium bicarbonate solution and brine and then dried over magnesium sulfate. After filtering, the filtrate was concentrated to give a crude product, which was then flash chromatographed with eluent (5%-30% ethyl acetate / dichloromethane) to afford the corresponding sulfoxide.

### Example 88.1

¹H NMR (DMSO): δ 11.75 (1H, s), 8.82 (1H, s), 8.68 (1H, s), 8.15-8.20 (2H, m), 8.09 (1H, d, *J=* 8.5 Hz), 7.85-7.91 (1H, m), 7.67-7.75 (3H, m), 7.57-7.64 (1H, m), 7.17 (2H, s). MS (M+H) 511. mp 239.5 °C with decomposition. Elemental Analysis: theory C 49.28, H 2.56, N 5.47; found C 49.30, H 2.63, N 5.37.

### Example 88.2

¹H NMR(DMSO): δ 11.5-12.0 (broad), 8.83 (1H, s), 8.68 (1H, s), 8.15-8.20 (2H, m), 8.09 (1H, d, *J=* 8.5 Hz), 7.85-7.92 (2H, m), 7.55-7.75 (2H, m), 7.17 (2H, s). MS (M-H) 542.9. mp: 234.4. Elemental Analysis: theory C 46.17, H 2.21, N 5.13; found C 45.97, H 2.26, N 4.92.

### Example 88.3

¹H NMR(DMSO) δ 11.43 (1H, s), 8.81 (1H, s), 8.68 (1H, s), 8.18 (1H, d, *J* = 8.2 Hz), 8.09 (1H, d, *J=* 8.5 Hz), 7.82-7.90 (3H, m), 7.58-7.74 (4H, m), 7.21 (2H, s). MS (M+H) 476.9. mp 261.8 °C with decomposition. Elemental Analysis: theory C 52.83, H 2.96, N 5.87; found C 52.71, H 3.05, N 5.71.

### EXAMPLE 174

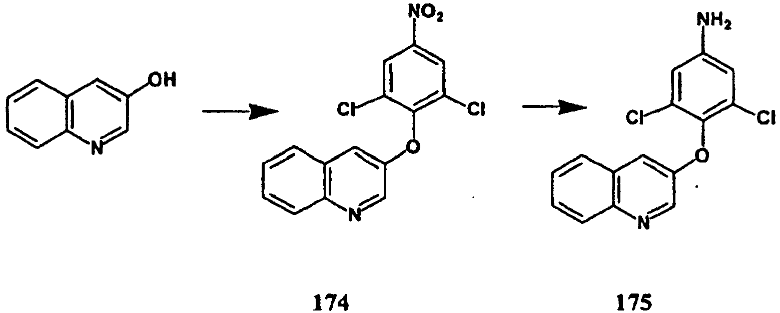

3-Hydroxyquinoline (prepared according to the procedure of Naumann, et. al., Synthesis, 1990, 4, 279-281)) (3 g) and 1,2,3- trichloro-5-nitrobenzene (4.7 g) were dissolved in DMF (80 mL) and heated with cesium carbonate (7.4g) for 2 hr at 60°C. The reaction was poured into ice/water (500 ml). The resulting off-white precipitate was collected by filtration and rinsed with hexane to afford compound **174** as a solid (6.9g) suitable for use in the next reaction.
¹H NMR in CDCl₃ 8.863 (d, J=2.2Hz, 1H), 8.360 (s, 2H), 8.106 (d, J=8.6Hz, 1H), 7.646 (m, 2H), 7.529 (d, J=8.6Hz, 1H), 7.160 (d, J=2.2Hz, 1H)

### EXAMPLE 175

To a solution of compound **180** (6.9 g) in ethanol/THF/water (ratio 40:20:10) was added ammonium chloride (3.3 g) and powdered iron (3.4g). This mixture was heated to reflux for 5 hr. The hot mixture was then filtered through Celite and concentrated. The residue was dissolved in ethyl acetate and washed with saturated NaHCO₃ solution followed by water and then brine. The solution was dried over magnesium sulfate and concentrated to afford compound **175** as an off-white solid (5.6 g).
¹H NMR in (DMSO) δ 8.846 (d, J=2.9Hz, 1H), 8.010 (m, 1H), 7.915 (m, 1H), 7.645 (m, 1H), 7.560 (m, 1H), 7.401 (d, J=2.9Hz, 1H), 6.778 (s, 2H), 5.762 (s, 2H).

Treatment of the aniline **175** with various sulfonyl chlorides according to conventional methods gave the sulfonamides of **Table 24.**

**Table 24**

| | | | | | | | |
|---|---|---|---|---|---|---|---|
| | | 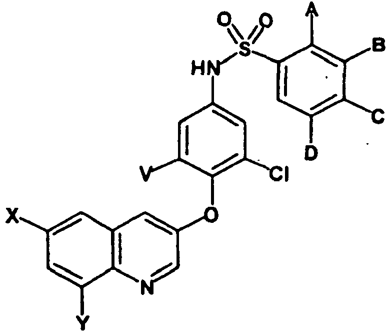 | | | | | |

| **Example** | **X** | **Y** | **V** | **A** | **B** | **C** | **D** |
|---|---|---|---|---|---|---|---|
| **176** | H | H | Cl | CF₃ | H | Cl | H |
| **177** | H | H | Cl | Cl | H | CF₃ | H |
| **178** | H | H | Cl | Cl | H | Cl | H |
| **180** | H | H | H | Cl | H | Cl | H |
| **181** | -CO₂Me | H | Cl | Cl | H | Cl | H |
| **182** | H | -CO₂Me | Cl | Cl | H | Cl | H |
| **183** | -CO₂H | H | Cl | Cl | H | Cl | H |
| **184** | H | -CO₂H | Cl | Cl | H | Cl | H |
| **185** | Me | H | Cl | Cl | H | Cl | Me |
| **186** | H | H | F | Cl | H | Cl | Me |

### EXAMPLE 176

¹H NMR (DMSO) δ 11.4-11.6 (1H, broad), 8.87 (1H, d, *J*= 2.9 Hz), 8.15-8.22 (2H, m), 8.00-8.08 (2H, m), 7.87 (1H, d, *J*= 8.0 Hz), 7.55-7.68 (2H, m), 7.47 (1H, d, *J*= 2.9 Hz), 7.35 (2H, s). MS (M-H) 545. mp 98.8 °C.

### EXAMPLE 177

¹H NMR(DMSO) δ 11.58 (1H, s), 8.86 (1H, d, *J*= 2.9 Hz), 8.38 (1H, d, *J* = 8.4 Hz), 8.23 (1H, s), 8.01 (1H, d, *J*= 8.4 Hz), 7.86 (1H, d, *J* = 8.1 Hz), 7.53-7.68 (2H, m), 7.46 (1H, d, *J*= 2.9 Hz), 7.34 (2H, s). MS (M-H) 545.0

### EXAMPLE 178

¹H NMR(d₆-acetone) 9.9 (1H, br s), 8.794 (1H, d, *J* = 2.9 Hz), 8.23 (1H, d, *J*= 8.4 Hz), 8.035 (1H, br d, *J*=8.4 Hz), 7.793 (1H, d, *J*= 1.5 Hz), 7.78 (1H, m), 7.62-7.70 (2H, m), 7.57 (1 H, td, *J* = 6.8,1.2 Hz), 7.476 (2H, s), 7.364 (1H, d, *J*=2.6 Hz). MS (M-H) 511.0.

### EXAMPLE 179

¹H NMR(300MHz/CDCl₃) δ 2.43(3H, s), 7.10(1H, d, J=3Hz), 7.26(2H, s ), 7.48-7.64(4H, m), 7.96(1H, s), 8.09(1H, d, J= 8.7Hz), 8.78(1H, d, J=3Hz). MS(M+H) 527. mp 233-235

### EXAMPLE 180

¹H NMR(300MHz/CDCl₃) δ 7.14(1H, dd, J=2.6Hz,J=8.9Hz), 7.26(1H, d, J=8.9Hz), 7.33(1H, d, J=2.6Hz), 7.56-7.58(2H, m), 7.66-7.69(2H,m), 7.87(1H, m), 7.93(1H, d, J=2.0Hz), 8.00(1H, m), 8.09(1H, d, J=8.5Hz), 8.80(1H, d, J=2.9Hz), 11.06(1H, brs), MS(M+H)) 479. mp 12: °C

### EXAMPLE 181

### 3-[2,6-Dichloro-4-(2,4-dichloro-benzenesulfonylamino)-phenoxy]-quinoline-6-carboxylic acid methyl ester (181)

A solution of 3-(4-Amino-2,6-dichloro-phenoxy)-quinoline-6-carboxylic acid methyl ester (312) (0.93mmol) and 2,4-dichlorobenzenesulfonyl chloride (250mg, 1.02mmol) in Pyridine (0.13ml, 1.53mmol)-CH₂Cl₂ (3.7ml) was stirred at room temperature for 12 hr. Sat NaHCO₃ was added to the reaction mixture, which was then extracted twice with AcOEt. Organic layer was washed by brine, dried over anhydrous MgSO₄, and concentrated. Crude residue was purified by column chromatography (Hexane/AcOEt=2/1, 80g of silica gel) to afford compound 181 (237mg, 41%, in 3 steps).
¹H NMR (300MHz,DMSO-d₆) δ 3.90 (3H, s), 7.31(2H, s), 7.72 (1H, dd, J=1.8, 7.8Hz), 7.79 (1H, d, J=3.0Hz), 7.96 (1H, d, J=1.8Hz), 8.11 (2H, s), 8.18 (1H, d, J=7.8Hz), 8.64 (1H, s), 8.99 (1H, d, J=3.0Hz), 11.42 (1H, br s). MS (M+H) 571

### EXAMPLE 182

### 3-[2,6-Dichloro-4- (2,4-dichloro-benzenesulfonylamino)-phenoxy]-quinoline-8-carboxylic acid methyl ester (182)

To a solution of 3-(4-Amino-2, 6-dichloro-phenoxy)-quinoline-8-carboxylic acid methyl ester (315) (1.26mmol) in Pyridine (0.15ml, 1.80mmol) and CH₂Cl₂ (5ml), was added 2,4-Dichlorolbenzenesulfonyl chloride (381mg, 1.55mmol). The mixture was stirred at room temperature for 12hr. Sat NaHCO₃ was added to the reaction mixture, which was then extracted twice with AcOEt. Organic layer was washed by Brine, dried over MgSO₄, and concentrated. The crude residue was purified by column chromatography (Hexane/AcOEt=2/1, 80g of silica gel) to afford compound 182 (506mg, 70%) as a white solid.
¹H NMR (300MHz,DMSO-d₆) δ 3.91 (3H, s), 7.31(2H, s), 7.57-7.65 (2H, m), 7.72 (1H, dd, J=2.1, 8.6Hz), 7.83(1H, d, J=8.6Hz), 7.96 (2H, d, J=2.1Hz), 8.03 (1H, d, J=8.6Hz), 8.18 (1H, d, J=8.6Hz), 8.94 (1H, d, J=2.1Hz), 11.4 (1H, br s), MS(M+H) 571

### EXAMPLE 183

### 3-[2,6-Dichloro-4-(2,4-dichloro-benzenesulfonylamino)-phenoxy]-quinoline-6-carboxylic acid (183)

To a solution of 3-[2,6-Dichloro-4- (2,4-dichloro-benzenesulfonylamino)-phenoxy]-quinoline-6-carboxylic acid methyl ester (181) (200mg, 0.35mmol) in THF/MeOH(2ml/2ml) was added 4N NaOH (0.1 ml, 0.4mmol). This mixture was refluxed for 2.5 hr. The reaction mixture was cooled to room temperature and was neutralized with 2N HCl, and then concentrated. The residue was extracted twice with AcOEt. Organic layer was washed by Brine, dried over anhydrous MgSO₄, and concentrated to give a solid. Crude product was recrystallized by Hexane/AcOEt to afford compound 183(153mg, 78%).
¹H NMR (300MHz,DMSO-d₆) δ 7.16 (2H, s), 7.62(1H, dd, J=2.0, 8.5H), 7.73 (1H, d, J=2.9Hz), 7.82 (1H, s), 8.08-8.11 (3H, m), 8.60 (1H, s), 8.95 (1H, d, J=2.9Hz), 13.2 (1H, br s), MS (M+H) 557. mp 228-2

### EXAMPLE 184

### 3-[2,6-Dichloro-4-(2,4-dichloro-benzenesulfonylamino)-phenoxy]-quinoline-8-carboxylic acid (184)

To a solution of 3-[2,6-Dichloro-4-(2-chloro-4-trifluoromethyl-benzenesulfonylamino)-phenoxy]-quinoline-8-carboxylic acid methyl ester (183) (402mg, 0.7mmol) in THF/MeOH=0.1ml/0.3ml was added 4N NaOH (0.2ml, 0.77mmol). The mixture was refluxed for 12hr. After cooling to room temp. the reaction mixture was filtered to remove insoluble materials. The filtrate was concentrated and the residue was dissolved in aq NH₄Cl and extracted twice with AcOEt. Organic layer was washed by Brine, and dried over anhydrous MgSO₄, and concentrated to afford compound 184 (197mg, 50%) as a white solid.
¹H NMR (300MHz,DMSO-d₆) δ 7.32 (2H, s), 7.70-7.81(2H, m), 7.90 (1H, d, J=2.2Hz), 7.96 (1H, d, J=2.2Hz), 8.17-8.19 (1H, m), 8.22-8.24 (1H, m), 8.38-8.39 (1H, m), 9.11 (1H, d, J=2.2Hz), 11.4 (1H, br s), 15.4 (1H, br s). MS (M+H) 557. mp 263-266 °C.

### EXAMPLE 185

### 2,4-Dichloro-N- [3,5-dichloro-4-(6-methyl-quinoln-3-yloxy)-phenyl]-5-methyl-benzenesulfonamide(185)

To a solution of 3,5-Dichloro-4- (6-methyl-quinlin-3-yloxy)-phenylamine (339) (400mg, 1.25mmol) in Pyridine (0.12ml, 1.48mmol)- CH₂Cl₂ (4ml) was added 2,4-Dichloro-5-methylbenzenesulfonyl chloride (325mg, 1.25mmol). The mixture was stirred at room temperature for 12hr. The reaction mixture was concentrated and the residue was purified by column chromatography (Hexane/AcOEt=2/1, 80g of silica gel) to provide compound (185) (453mg, 66%) as a white solid.
¹H NMR (300MHz,DMSO-d₆) δ 2.41 (3H, s), 2.44(3H, s), 7.31 (3H, s), 7.49 (1H, d, J=8.7Hz), 7.61 (1H, s), 7.88-7.91 (2H, m), 8.19 (1H, s), 8.74 (1H, d, J=3.0Hz), 11.3 (1H, br s), MS (M+H) 541 mp 228-230°C.

### EXAMPLE 186

### PART 1

Preparation of 3-chloro-5-fluoro-4-(quinolin-3-yloxy)nitrobenzene (186.1) To a solution of 3,4-Difluoronitrobenzene 1.00g in conc.H₂SO₄ (20ml), was added portionwise Cl₂O in CCl₄(25ml, prepared as described by Cady G. H. et. al in Inorg. Synth. Vol 5, p156(1957)). The mixture was stirred at room temperature overnight. The mixture was poured into crashed ice and extracted with Et₂O (30mlx3). Combined ether layers were washed with 10%Na₂SO₃ and brine, and dried over Na₂SO₄. The solvent was concentrated to Ca. 10ml(This solution contains 3-Chloro-4,5-difluoronitrobenzene). This solution was diluted with acetone (60ml), and then 3-hydroxyquinoline 0.75g and K₂CO₃ 2.2g were added to this solution. The mixture was heated to reflux for 1.5 hr. After cooling the reaction mixture was filtered through a short celite pad. The filtrate was concentrated to give an foil, which was then purified by column chromatography (silica gel, AcOEt:Hexane=1:5) to provide the intermediate compound 186.1 (0.980g) as a yellow oil.

### PART 2

### Preparation of 3-Chloro-5-fluoro-4-(quinolin-3-yloxy)phenylamine (186.2)

To a solution of 3-Chloro-5-fluoro-4-(quinolin-3-yloxy)nitrobenzene (186.1) (0.980g) and NH₄Cl (1.64g) in EtOH(50ml) - H₂O (5ml), was added iron powder (1.92g). The mixture was heated to reflux for 1hr. After cooling the reaction mixture was filtered through short celite pad. The filtrate was concentrated, diluted with sat. NaHCO₃ and extacted with AcOEt(30mlx3). The combined organic layeres were washed with brine and dried over Na₂SO₄. Concentration of solvent afford crude product, which was purified by column chromatography (silicagel, AcOEt:Hexane=1:3) to provide aniline 186.2 (0.420g) as a colorless solid.

### PART 3

### Preparation of N-[3-chloro-5-fluoro-4-(quinolin-3-yloxy)phenyl]-2,4-dichloro-5-methyl-benzenesulfonamide (186)

To a solution of 3-chloro-5-fluoro-4-(quinolin-3-yloxy)phenylamine (186.2) (0.420g) in pyridine(2.2ml), was added 2,4-dichloro-5-methylbenzenesulfonylchloride 0.360g. The mixture was stirred at room for 1hr. The reaction mixture was purified directly by column chromatography (silicagel, AcOEt:Hexane=1:3). The product was triturated by hexane to give title compound (0.522g). (73%) as a solid.
NMR(300MHz/CDCl₃) δ 2.43(3H, s), 7.05(1H, d, J=2.6Hz), 7.09-7.11(1H, m), 7.21(1H, d, J=2.6Hz), 7.36(1H, brs), 7.49-7.66(4H, m), 7.96(1H, s), 8.10(1H, d, J=8.2Hz), 8.80(1H, brs). MS (M+H) 511, mp 187 °C.

### EXAMPLE 308

### 3-Hydroxy-6-methylquinoline (308)

A solution of 3-Amino-6-methylquinoline [(1.21g, 7.65mmol), prepared according to J.Chem.Soc.2024-2027(1948) Morley, J. S.; Simpson, J. C. E.] in 6N H₂SO₄ (25ml) was cooled in an ice bath. To the solution NaNO₂ (560mg, 8.10mmol) in water (2ml) was added and stirred for 30min at 0 degrees. Separately 5% H₂SO₄ was refluxed and above Diazo reaction mixture was added to this refluxing solution. After 30min the reaction mixture was cooled to room temperature, and was neutralized by 6N NaOH. The resulting insoluble material was collected by filtration. This solid was recrystallized by CHCl₃/AcOEt to afford compound (308) (348mg, 29%).
¹H NMR (300MHz,DMSO-d₆) δ 7.34 (1H, dd, J=1.9, 8.6Hz), 7.42(1H, d, J=2.8Hz), 7.55 (1H, s), 7.79 (1H, d, J=8.6Hz), 8.50 (1H, d, J=2.8Hz).

### EXAMPLE 309

### 3-(2,6-Dichloro-4-nitro-phenoxy)-6-methyl-quinoline (309)

To a solution of 3-Hydroxy-6-methylquinoline (308) (348mg, 2.19mmol) in DMF (3.5ml), was added NaH (60% oil suspension, 90mg, 2.25mmol) in one portion at room temperature. After 5min 3,4,5-Trichloronitorobenzene (509mg, 2.25mmol) in DMF (2ml) was added and the reaction mixture was heated at 50 degrees with stirring for 2hr. After cooling to room temperature. Ice/water was added to the reaction mixture, which was then acidified with 2N HCl and extracted twice with AcOEt. Organic layer was washed with Brine, dried over anhydrous MgSO₄, and concentrated. Crude residue was purified by column chromatography (Hexane/AcOEt=4/1,80g of silica gel) to afford compound 309 (510mg, 67%).
¹H NMR (300MHz,DMSO-d₆) δ 7.52-7.57(2H,m), 7.61 (1H, s), 7.94(1H, d, J=8.6Hz), 8.63 (2H, s), 8.86 (1H, d, J=2.9Hz).

### EXAMPLE 310

### 3-(2,6-Dichloro-4-nitro-phenoxy)-quinoline-6-carboxylic acid (310).

A solution of 3-(2,6-Dichloro-4-nitro-phnoxy)-6-methyl-quinoline(309) (510mg, 1.46mmol) and chromium (VI) oxide (292mg, 2.92mmol) in c H₂SO₄/ H₂O =2.4ml/4.7ml was heated at 100 degrees while three 292mg portions of chromic anhydride were added eight hour intervals. After 32hr heating was stopped and allowed to stand for over night. Insoluble material was collected by filtration, and this solid was washed with water twice to afford compound (310)(443mg, 80%).
¹H NMR (300MHz,DMSO-d₆) δ 7.94 (1H, d, J=3.0Hz), 8.14(2H, s), 8.56 (1H, s), 8.65 (2H, s), 9.09 (1H, d, J=3.0Hz).

### EXAMPLE 311

### 3-(2,6-Dichloro-4-nitro-phenoxy)-quinoline-6-carboxylic acid methyl ester (311)

To a solution of 3-(2,6-Dichloro-4-nitro-phenoxy)-quinoline-6-carboxylic acid (310) (443mg, 0.93mmol) in dry THF (20ml) was added CH₂N₂ in Et₂O solution [Prepared from Nitrosomethylurea (1.65g) and 50%KOH (5ml)]. This mixture was stirred at room temperature for 1hr. AcOH (1ml) was added to the reaction mixture, which was then concentrated. Sat NaHCO₃ was added to the residue, which was extracted twice with AcOEt. Organic layer was washed by Brine, dried over anhydrous MgSO₄, and concentrated to afford compound 311 (415mg).
¹H NMR (300MHz,DMSO-d₆) δ 3.89 (3H, s), 5.75(2H, br s), 6.76 (2H, s), 7.73 (1H, d, J=2.9Hz), 8.09 (2H, s), 8.67 (1H, s), 8.94 (1 H, d, J=2.9Hz).

### EXAMPLE 312

### 3-(4-Amino-2,6-dichloro-phenoxy)-quinoline-6-carboxylic acid methyl ester (312)

To a solution of 3-(2,6-Dichloro-4-nitro-phenoxy)-quinoline-6-carboxylic acid methyl ester (311) (0.93mmol) and NH₄Cl (283mg, 5.3mmol) in EtOH/THF/water (8ml/16ml/1ml )was added Iron powder (296mg, 5.3mmol). The reaction mixture was refluxed for 4hr. Insoluble materials were removed by Celite pad, which was washed by THF, acetone and then EtOH. The filtrate was concentrated, and sat NaHCO₃ was added and extracted twice with AcOEt. Organic layer was washed by brine, dried over anhydrous MgSO₄, and concentrated to afford compound 312 (372mg, over weight).
¹H NMR (300MHz,DMSO-d₆) δ 3.89 (3H, s), 5.75(2H,s), 6.76 (2H, s), 7.73 (1H, d, J=2.9Hz), 8.09 (2H, s), 8.67 (1H, s), 8.94 (1H, d, J=2.9Hz).

### EXAMPLE 313

### 3-Hydroxy-8-quinolinecarboxylic acid methyl ester (313)

To the mixture of 8-Quinoline carboxylic acid (500mg, 2.89mmol) in THF (80ml) was added CH₂N₂ in Et₂O sol. [Prepared from Nitrosomethylurea (1.65g) and 50%KOH (5ml)] at room temperature. The reaction mixture was stirred for 12 hr and then concentrated to give the intermediate ester.
¹H NMR (300MHz,DMSO-d₆) δ 3.92 (3H, s), 7.60-7.70 (2H, m), 7.93-7.96(1H, m), 8.14-8.17 (1H, m), 8.44-8.48(1H, m), 8.97-8.99(1H, m)

To a solution of the intermediate 8-Quinolinecarboxylic acid methyl ester (2.89mmol) in AcOH (4ml) was added 30% H₂O₂ (0.6ml). The reaction mixture was heated at 85 degrees for 7.5hr. The reaction mixture was treated with sat NaHCO₃, and extracted six times with CHCl₃. Organic layer was dried over anhydrous MgSO₄, and concentrated. Crude residue was triturated with CHCl₃/Toluene to provide compound 313 (256mg, 44%, in 2 steps).
¹H NMR (300MHz,DMSO-d₆) δ 3.89 (3H, s), 7.52(1H, d, J=6.9Hz), 7.57 (1H, d, J=1.5Hz), 7.66 (1H, dd, J=1.5,6.9Hz), 7.95 (1H, dd, J=1.5, 8.1Hz), 8.63 (1H, d, J=2.7Hz), 10.5 (1H, br s).

### EXAMPLE 314

### 3-(2,6-Dichloro-4-nitro-phenoxy)-quinoline-8-carboxylic acid methyl ester (314)

To a solution of 3-Hydroxy-8-quinolinecarboxylic acid methyl ester (313) (256mg, 1.26mmol) and 3,4,5-Trichloronitrobenzene (294mg, 1.30mmol) in Acetone (40ml) was added K₂CO₃ (870mg, 6.30mmol). This mixture was refluxed for 3.5hr. The reaction mixture was cooled to room temperature and insoluble materials were removed by Celite filtration. The filtrate was concentrated and the residue was purified by column chromatography. (Hexane/AcOEt=4/1, 80g of silica gel) to afford compound 314.
¹H NMR (300MHz,DMSO-d₆) δ 3.92 (3H, s), 7.67(1H, dd, J=7.3Hz), 7.79 (1H, d, J=2.9Hz), 7.88 (1H, dd, J=1.5, 7.3Hz), 9.05 (1H, d, J=2.9Hz).

### EXAMPLE 315

### 3-(4-Amino-2,6-dichloro-phenoxy)-quinoline-8-carboxylic acid methyl ester(315).

To a solution of 3-(2,6-Dichloro-4-nitro-phenoxy)-quinoline-8-carboxylic acid methyl ester (314) (1.26mmol) and NH₄Cl (370mg, 6.91 mmol) in EtOH/THF/H₂O =8ml/4ml/2ml was added Iron powder (386mg, 6.91mmol). The reaction mixture was.refluxed for 3.5hr. After cooling to room temperature and insoluble materials were filtered by Celite filtration. The filtrate was concentrated and sat NaHCO₃ was added to the residue, which was extracted twice with AcOEt. Organic layer was washed by Brine, dried over MgSO₄, and concentrated. Crude residue was purified by column chromatography (Hexane/AcOEt=2/1, 80g of silica gel) to afford compound 315 (543mg).
¹H NMR (300MHz,DMSO-d₆) δ 3.91 (3H, s), 5.77(2H, br s), 6.78 (2H, s), 7.50 (1H, d, J=3.0Hz), 7.61 (1H, dd, J=8.1Hz), 7.81 (1H, dd, J=1.4, 6.4Hz), 8.08 (1H, dd, J=1.4Hz, 6.4Hz), 8.93 (1H, d, J=3.0Hz).

### EXAMPLE 339

### 3,5-Dichloro-4-(6-methyl-quinolin-3-yloxy)-phenylamine (339)

To a solution of 3-(2,6-Dichloro-4-nitro-phenoxy)-6-methyl-quinoline (309) (1.30g, 3.71mmol) and NH₄Cl (992mg, 18.55mmol) in EtOH/THF/H₂O =12ml/12ml/3ml, was added Iron Powder (1.04g, 18.55mmol). The mixture was refluxed for 4 hr. Insoluble materials were removed by Celite filtration. The filtrate was concentrated and sat NaHCO₃ was added to the residue, which was then extracted twice with AcOEt. Organic layer was washed with Brine, dried over anhydrous MgSO₄, and concentrated to afford compound 339 (1.18g, 98%).
¹H NMR (300MHz,DMSO-d₆) δ 2.44 (3H, s), 5.75 (2H, br s), 6.77 (2H, s), 7.27 (1H, d, J=2.8Hz), 7.48 (1H, d, J=8.6Hz), 7.67 (1H, s), 7.89 (1H, d, J=8.6Hz), 8.74 (1H, d, J=2.8Hz).

### EXAMPLE 373

Using methods similar to Lehmann, *et al., ibid.,* selected compounds exhibited the following IC₅₀ values in a PPARγ ligand binding assay utilizing [³H]-BRL 49653 as the radioligand. IC₅₀ values are defined as the concentration of test compounds required to reduce by 50% the specific binding of [³H]-BRL 49653 and are represented by (+) <30 µM; (++) < 10 µM; (+++) < 1 µM.

**TABLE 38**

| **Compound** | **IC₅₀(µM)** |
|---|---|
| **86** | +++ |
| **87.3** | +++ |
| **178** | +++ |
| **179** | +++ |

### EXAMPLE 374

Selected compounds were administered to KK-Ay mice as a 0.018% (30 mg/kg) dietary admixture in powdered diet and evaluated for anti-diabetic efficacy as described (T. Shibata, K. Matsui, K. Nagao, H. Shinkai, F. Yonemori and K. Wakitani 1999; European Journal of Pharmacology 364:211-219). The change in serum glucose levels compared to untreated control animals is exemplified in Table 39.

**TABLE 39**

| **Example #** | **KKAy Glucose** |
|---|---|
| **87.3** | ++ |
| **178** | ++ |
| **179** | ++ |

| | |
|---|---|
| (-) <10%; (+) 10% to 20%; (++) glucose lowering >20%. | |

## Claims

1. A compound having the formula: 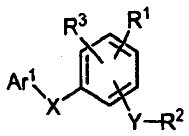 or a pharmaceutically acceptable salt thereof, wherein
Ar¹ is a substituted or unsubstituted 3-quinolinyl;
X is -O-, -NH-, or -S-;
Y is -NH-S(O)₂-;
R¹ is a member selected from the group consisting of halogen, (C₁-C₈)alkyl, (C₁-C₈)alkoxy, -C(O)R¹⁴, -CO₂R¹⁴, and -C(O)NR¹⁵R¹⁶, wherein
R¹⁴ is a member selected from the group consisting of hydrogen, (C₁-C₈)alkyl, (C₂-C₈)heteroalkyl; wherein heteroalkyl means a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms are optionally oxidized and the nitrogen atom may be optionally quarternized, the heteroatoms 0, N and S may be placed at any interior position of the heteroalkyl group; aryl, and aryl(C₁-C₄)alkyl;
R¹⁵ and R¹⁶ are members independently selected from the group consisting of hydrogen, (C₁-C₈)alkyl, (C₂-C₈)heteroalkyl; wherein heteroalkyl means a stable straight or branched chain, or cyclic hydrocarbon radical, or combinations thereof, consisting of the stated number of carbon atoms and from one to three heteroatoms selected from the group consisting of O, N, Si and S, and wherein the nitrogen and sulfur atoms are optionally oxidized and the nitrogen atom may be optionally quarternized, the heteroatoms 0, N and S may be placed at any interior position of the heteroalkyl group; aryl, and aryl(C₁-C₄)alkyl, or taken together with the nitrogen to which each is attached form a 5-, 6-, or 7-membered ring;
R² is a phenyl group having from 0 to 3 substituents selected from the group consisting of halogen, -OCF₃, -OH, -O(C₁-C₈)alkyl, -C(O)-(C₁-C₈)alkyl, -CN, -CF₃, (C₁-C₈)alkyl, and -NH₂; and
R³ is a member selected from the group consisting of halogen, methoxy and trifluoromethoxy.

2. A compound of claim 1, represented by a formula selected from the group consisting of 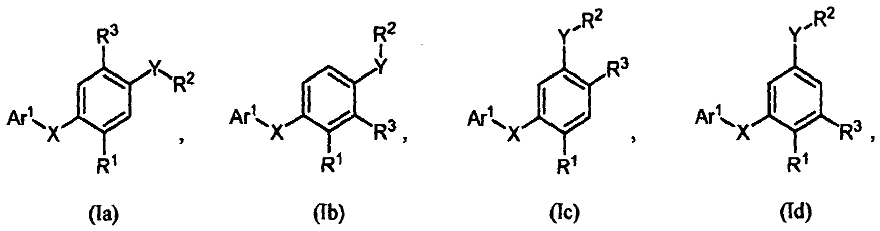 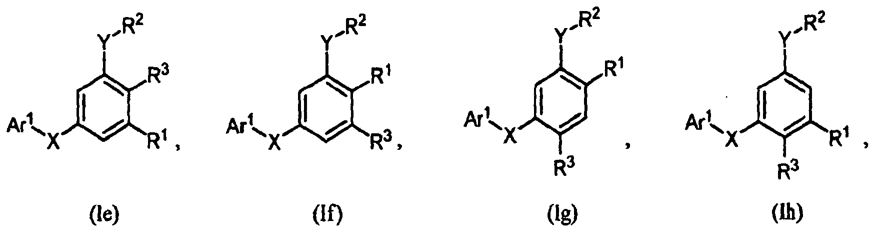 and

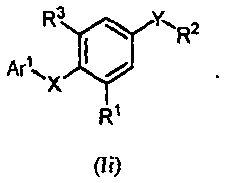

3. A compound of claim 2, represented by the formula

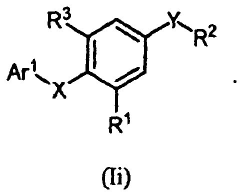

4. A compound of claim 3, wherein R¹ is a member selected from the group consisting of halogen, (C₁-C₈)alkyl, and (C₁-C₈)alkoxy; R² is a phenyl group having from 0 to 3 substituents selected from the group consisting of halogen, -OCF₃, -OH, -O(C₁-C₈)alkyl, -C(O)-(C₁-C₈)alkyl, -CN, -CF₃, (C₁-C₈)alkyl, and -NH₂; and R³ is selected from the group consisting of halogen, methoxy and trifluoromethoxy.

5. A compound of claim 4, wherein R² is a phenyl group having from 0 to 3 substituents selected from the group consisting of halogen, -OCF₃, and -CF₃.

6. A compound of claim 5, wherein R¹ and R³ are each independently a halogen and R² is a phenyl group having from 0 to 3 substituents selected from the group consisting of halogen, -OCF₃, and -CF₃.

7. A compound of claim 1, wherein the compound is of the formula

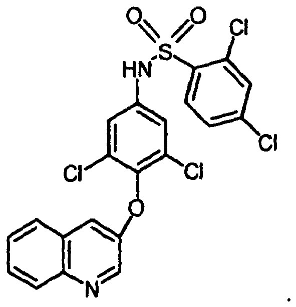

8. The compound of claim 1, wherein the quinolinyl is unsubstituted.

9. A composition comprising a pharmaceutically acceptable excipient and a compound of any one of the preceding claims.

10. The use of a compound of any one of claims 1 to 8 for the manufacture of a medicament for use in modulating a metabolic disorder or inflammatory condition in a host.

11. A compound of any one of claims 1 to 8 for use in modulating a metabolic disorder or inflammatory condition in a host.

12. The use of claim 10, or the compound for use in modulating a metabolic disorder or inflammatory condition in a host of claim 11, wherein said host is a mammal selected from the group consisting of humans, dogs, monkeys, mice, rats, horses and cats.

13. The use of claim 10 or 12, or the compound for use in modulating a metabolic disorder or inflammatory condition in a host of claims 11 or 12, wherein said compound is formulated for oral administration.

14. The use of claim 10 or 12, or the compound for use in modulating a metabolic disorder or inflammatory condition in a host of claims 11 or 12, wherein said compound is formulated for topical administration.

15. The use of claims 10, 12, 13 or 14, or the compound for use in modulating a metabolic disorder or inflammatory condition in a host of claims 11, 12, 13 or 14, wherein said modulating prevents a PPARγ-mediated condition.

16. The use of claims 10, 12, 13 or 14, or the compound for use in modulating a metabolic disorder or inflammatory condition in a host of claims 11, 12, 13 or 14, wherein said disorder or condition is selected from the group consisting of NIDDM, obesity, and hypercholesterolemia and other lipid-mediated diseases, and inflammatory conditions.

17. The use of claims 10, 12, 13 or 14, or the compound for use in modulating a metabolic disorder or inflammatory condition in a host of claims 11, 12, 13 or 14, wherein said compound is formulated for parenteral administration.

18. The use of claim claims 10, 12, 13 or 14, or the compound for use in modulating a metabolic disorder or inflammatory condition in a host of claims 11, 12, 13 or 14, wherein said metabolic disorder is mediated by PPARγ.

19. The use of claims 10, 12, 13 or 14, or the compound for use in modulating a metabolic disorder or inflammatory condition in a host of claims 11, 12, 13 or 14, wherein said metabolic disorder is NIDDM.

20. The use of claims 10, 12, 13 or 14, or compound for use in modulating a metabolic disorder or inflammatory condition in a host of claims 11,12,13 or 14, wherein said inflammatory condition is rheumatoid arthritis or atherosclerosis.

21. The use of claims 10 or 12 to 20, or compound for use in modulating a metabolic disorder or inflammatory condition in a host of claims 11, or 12 to 20, wherein the host is human.

## Patentansprüche

1. Verbindung mit der Formel: 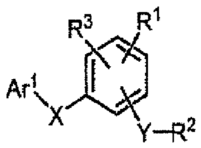 oder einem pharmazeutisch annehmbaren Salz derselben, wobei
Ar¹ ein substituiertes oder unsubstituiertes 3-Chinolinyl ist;
X -O-, -NH- oder-S- ist;
Y -NH-S(O)₂- ist;
R¹ ein Element ist, das aus der Gruppe ausgewählt ist, die aus Halogen, (C₁-C₈)-Alkyl, (C₁-C₈)-Alkoxy, -C(O)R¹⁴, -CO₂R¹⁴ und -C(O)NR¹⁵R¹⁶ besteht, wobei
R¹⁴ ein Element ist, das aus der Gruppe ausgewählt ist, die aus Wasserstoff, (C₁-C₈)-Alkyl, (C₂-C₈)-Heteroalkyl; wobei Heteroalkyl eine stabile gerade oder verzweigte Kette bedeutet, oder ein zyklisches Kohlenwasserstoffradikal oder Kombinationen davon, bestehend aus der angegebenen Anzahl von Kohlenstoffatomen und aus einem bis drei Heteroatomen, die aus der Gruppe ausgewählt sind, die aus O, N, Si und S besteht, und wobei die Stickstoff- und Schwefelatome gegebenenfalls oxidiert sind und das Stickstoffatom gegebenenfalls quaternisiert sein kann, die Heteroatome O, N und S in irgendeiner inneren Position der Heteroalkylgruppe platziert sein können; Aryl, und Aryl-(C₁-C₄)-alkyl besteht;
R¹⁵ und R¹⁶ Elemente sind, die unabhängig aus der Gruppe ausgewählt sind, die aus Wasserstoff, (C₁-C₈)-Alkyl, (C2-C₈)-Heteroalkyl; wobei Heteroalkyl eine stabile gerade oder verzweigte Kette bedeutet, oder ein zyklisches Kohlenwasserstoffradikal oder Kombinationen davon, bestehend aus der angegebenen Anzahl von Kohlenstoffatomen und aus einem bis drei Heteroatomen, die aus der Gruppe ausgewählt sind, die aus O, N, Si und S besteht, und wobei die Stickstoff- und Schwefelatome gegebenenfalls oxidiert sind und das Stickstoffatom gegebenenfalls quaternisiert sein kann, die Heteroatome O, N und S in irgendeiner inneren Position der Heteroalkylgruppe platziert sein könnten; Aryl und Aryl (C₁-C₄)-alkyl, oder zusammen mit dem Stickstoff genommen, an denen jeweils ein 5-, 6-oder 7-gliedriger Ring angebracht ist, besteht;
R² eine Phenylgruppe mit 0 bis 3 Substituenten ist, die aus der Gruppe ausgewählt sind, die aus Halogen, -OCF₃, -OH, -O(C₁-C₈)-Alkyl, -C(O)-(C₁-C₈)-Alkyl, -CN, -CF₃, (C₁-C₈)-Alkyl und -NH₂ besteht; und
R³ ein Element ist, das aus der Gruppe ausgewählt ist, die aus Halogen, Methoxy und Trifluormethoxy besteht.

2. Verbindung nach Anspruch 1, die durch eine Formel dargestellt ist, die aus der Gruppe ausgewählt ist, die aus 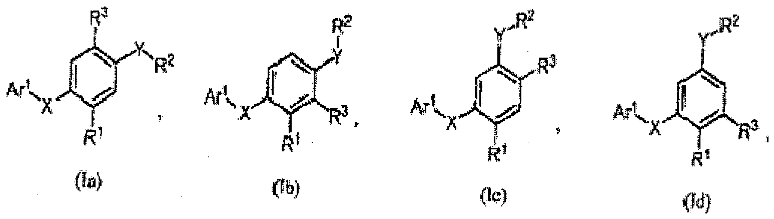 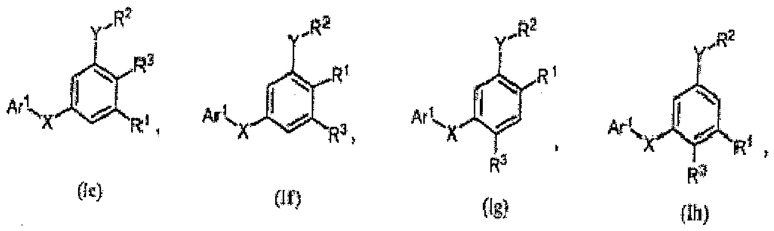 und 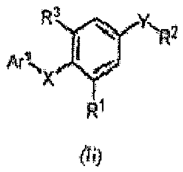 besteht.

3. Verbindung nach Anspruch 2, die durch die Formel 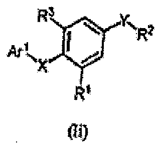 dargestellt ist.

4. Verbindung nach Anspruch 3, wobei R¹ ein Element ist, das aus der Gruppe ausgewählt ist, die aus Halogen, (C₁-C₈)-Alkyl und (C₁-C₈)-Alkoxy besteht; R² für eine Phenylgruppe steht, die 0 bis 3 Substituenten aufweist, die aus der Gruppe ausgewählt sind, die aus Halogen, -OCF₃, -OH, -O(C₁-C₈)-Alkyl, -C(O)-(C₁-C₈)-Alkyl, -CN, -CF₃, (C₁-C₈)-Alkyl und -NH₂ besteht; und R³ aus der Gruppe ausgewählt ist, die aus Halogen, Methoxy und Trifluormethoxy besteht.

5. Verbindung nach Anspruch 4, wobei R² eine Phenylgruppe mit 0 bis 3 Substituenten ist, die aus der Gruppe ausgewählt sind, die aus Halogen, -OCF₃ und -CF₃ besteht.

6. Verbindung nach Anspruch 5, wobei R¹ und R³ jeweils unabhängig ein Halogen sind und R² eine Phenylgruppe mit 0 bis 3 Substituenten ist, die aus der Gruppe ausgewählt sind, die aus Halogen, -OCF₃ und -CF₃ besteht.

7. Verbindung nach Anspruch 1, wobei die Verbindung von der Formel 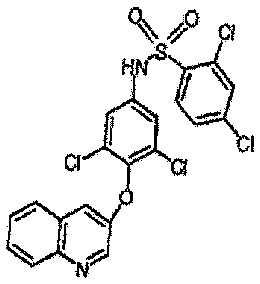 ist.

8. Verbindung nach Anspruch 1, wobei das Chinolinyl unsubstituiert ist.

9. Zusammensetzung, umfassend einen pharmazeutisch annehmbaren Arzneimittelträger und eine Verbindung nach irgendeinem der vorhergehenden Ansprüche.

10. Verwendung einer Verbindung nach irgendeinem der Ansprüche 1 bis 8 für die Herstellung eines Arzneimittels zur Verwendung bei der Modulation einer Stoffwechselstörung oder eines entzündlichen Leidens in einem Wirt.

11. Verbindung nach irgendeinem der Ansprüche 1 bis 8 zur Verwendung bei der Modulation einer Stoffwechselstörung oder eines entzündlichen Leidens in einem Wirt.

12. Verwendung nach Anspruch 10 oder die Verbindung zur Verwendung bei der Modulation einer Stoffwechselstörung oder eines entzündlichen Leidens in einem Wirt nach Anspruch 11, wobei der Wirt ein Säugetier ist, das aus der Gruppe ausgewählt ist, die aus Menschen, Hunden, Affen, Mäusen, Ratten, Pferden und Katzen besteht.

13. Verwendung nach Anspruch 10 oder 12 oder die Verbindung zur Verwendung bei der Modulation einer Stoffwechselstörung oder eines entzündlichen Leidens in einem Wirt nach den Ansprüchen 11 oder 12, wobei die Verbindung für eine orale Verabreichung formuliert ist.

14. Verwendung nach Anspruch 10 oder 12, oder die Verbindung zur Verwendung bei der Modulation einer Stoffwechselstörung oder eines entzündlichen Leidens in einem Wirt nach den Ansprüchen 11 oder 12, wobei die Verbindung für eine topische Verabreichung formuliert ist.

15. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei die Modulation ein PPARγ-vermitteltes Leiden verhindert.

16. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei die Störung oder das Leiden aus der Gruppe ausgewählt ist, die aus NIDDM, Adipositas und Hypercholesterinämie und anderen lipid-vermittelten Erkrankungen sowie entzündlichen Leiden besteht.

17. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei die Verbindung für eine parenterale Verabreichung formuliert ist.

18. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei die metabolische Störung durch PPARγ vermittelt wird.

19. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei die metabolische Störung NIDDM ist.

20. Verwendung nach den Ansprüchen 10, 12, 13 oder 14, oder die Verbindung zur Verwendung bei der Modulation einer Stoffwechselstörung oder eines entzündlichen Leidens in einem Wirt nach den Ansprüchen 11, 12, 13 oder 14, wobei das entzündliche Leiden Gelenkrheumatismus oder Atherosklerose ist.

21. Verwendung nach den Ansprüchen 10 oder 12 bis 20, oder die Verbindung zur Verwendung bei der Modulation einer Stoffwechselstörung oder eines entzündlichen Leidens in einem Wirt nach den Ansprüchen 11 oder 12 bis 20, wobei der Wirt ein Mensch ist.

## Revendications

1. Composé ayant la formule: 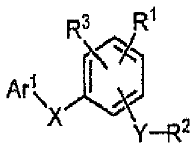 ou sel pharmaceutiquement acceptable dudit composé,
dans lequel
Ar¹ est un 3-quinolinyle substitué ou non substitué ;
X est -O-, -NH- ou-S- ;
Y est -NH-S(O)₂₋- ;
R¹ est un membre sélectionné dans le groupe constitué d'un halogène, d'un alkyle en C₁-C₈, d'un alcoxy en C₁-C₈, d'un radical -C(O)R¹⁴, d'un radical -CO₂R¹⁴ et d'un radical -C(O)NR¹⁵R¹⁶, dans lequel
R¹⁴ est un membre sélectionné dans le groupe constitué de l'hydrogène, d'un alkyle en C₁-C₈, d'un hétéroalkyle en C₂-C₈ ; dans lequel hétéroalkyle désigne une chaîne stable droite ou ramifiée, ou un radical d'hydrocarbure cyclique, ou des combinaisons de ceux-ci, comprenant le nombre indiqué d'atomes de carbone et de un à trois hétéroatomes sélectionnés dans le groupe constitué de O, N, Si et S, et dans lequel les atomes d'azote et de soufre sont éventuellement oxydés et l'atome d'azote peut être éventuellement quaternisé, les hétéroatomes O, N et S pouvant être placés à n'importe quelle position intérieure du groupe hétéroalkyle ; d'un aryle et d'un (alkyle en C₁-C₄)aryle ;
R¹⁵ et R¹⁶ sont des membres sélectionnés indépendamment dans le groupe constitué de l'hydrogène, d'un alkyle en C₁-C₈, d'un hétéroalkyle en C₂-C₈ ; dans lequel hétéroalkyle désigne une chaîne stable droite ou ramifiée, ou un radical d'hydrocarbure cyclique, ou des combinaisons de ceux-ci, comprenant le nombre indiqué d'atomes de carbone et de un à trois hétéroatomes sélectionnés dans le groupe constitué de O, N, Si et S, et dans lequel les atomes d'azote et de soufre sont éventuellement oxydés et l'atome d'azote peut être éventuellement quaternisé, les hétéroatomes O, N et S pouvant être placés à n'importe quelle position intérieure du groupe hétéroalkyle ; d'un aryle et d'un (alkyle en C₁-C₄)aryle ; ou sont pris ensemble avec l'azote auquel chacun est lié pour former un anneau à 5, 6 ou 7 membres ;
R² est un groupe phényle ayant de 0 à 3 substituants sélectionnés dans le groupe constitué d'un halogène, d'un radical -OCF₃, d'un radical -OH, d'un -O-alkyle en C₁-C₈, d'un -C(O)-alkyle en C₁-C₈, d'un radical -CN, d'un radical -CF₃, d'un alkyle en C₁-C₈ et d'un radical -NH₂ ; et
R³ est un membre sélectionné dans le groupe constitué d'un halogène, d'un méthoxy et d'un trifluorométhoxy.

2. Composé selon la revendication 1, représenté par une formule sélectionné dans le groupe constitué de 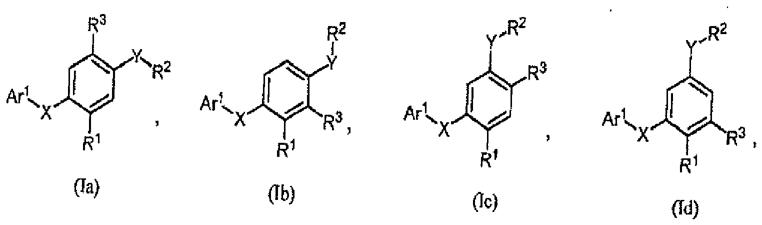 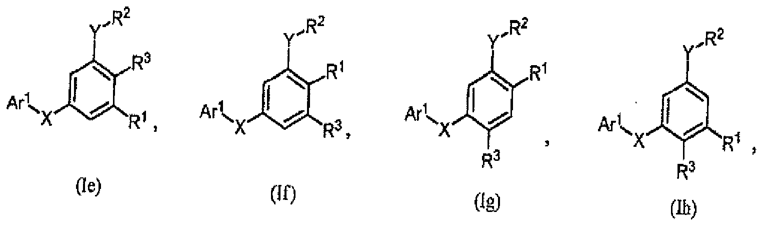 and

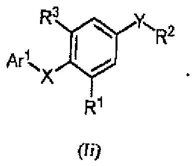

3. Composé selon la revendication 2, représenté par la formule

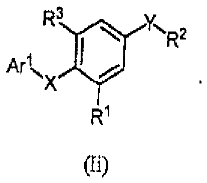

4. Composé selon la revendication 3, dans lequel R¹ est un membre sélectionné dans le groupe constitué d'un halogène, d'un alkyle en C₁-C₈ et d'un alcoxy en C₁-C₈ ; R² est un groupe phényle ayant de 0 à 3 substituants sélectionnés dans le groupe constitué d'un halogène, d'un radical -OCF₃, d'un radical -OH, d'un -O-alkyle en C₁-C₈), d'un -C(O)-alkyle en C₁-C₈, d'un radical -CN, d'un radical -CF₃, d'un alkyle en C₁-C₈ et d'un radical -NH₂ ; et R³ est sélectionné dans le groupe constitué d'un halogène, d'un méthoxy et d'un trifluorométhoxy.

5. Composé selon la revendication 4, dans lequel R² est un groupe phényle ayant de 0 à 3 substituants sélectionnés dans le groupe constitué d'un halogène, d'un radical-OCF₃ et d'un radical -CF₃.

6. Composé selon la revendication 5, dans lequel R¹ et R³ sont chacun indépendamment un halogène, et R² un groupe phényle ayant de 0 à 3 substituants sélectionnés dans le groupe constitué d'un halogène, d'un radical -OCF₃ et d'un radical -CF₃.

7. Composé selon la revendication 1, dans lequel le composé est de formule

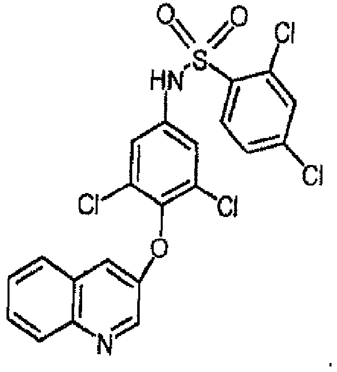

8. Composé selon la revendication 1, dans lequel le quinolinyle est non substitué.

9. Composition comprenant un excipient pharmaceutiquement acceptable et un composé selon l'une quelconque des revendications précédentes.

10. Utilisation d'un composé selon l'une quelconque des revendications 1 à 8 pour la fabrication d'un médicament destiné à être utilisé dans la modulation d'un trouble du métabolisme ou d'un état inflammatoire chez un hôte.

11. Composé selon l'une quelconque des revendications 1 à 8 destiné à être utilisé dans la modulation d'un trouble du métabolisme ou d'un état inflammatoire chez un hôte.

12. Utilisation selon la revendication 10, ou composé destiné à être utilisé dans la modulation d'un trouble du métabolisme ou d'un état inflammatoire chez un hôte selon la revendication 11, dans laquelle ou lequel ledit hôte est un mammifère sélectionné dans le groupe constitué des humains, des chiens, des singes, des souris, des rats, des chevaux et des chats.

13. Utilisation selon la revendication 10 ou 12, ou composé destiné à être utilisé dans la modulation d'un trouble du métabolisme ou d'un état inflammatoire chez un hôte selon la revendication 11 ou 12, dans laquelle ou lequel ledit composé est formulé pour une administration orale.

14. Utilisation selon la revendication 10 ou 12, ou composé destiné à être utilisé dans la modulation d'un trouble du métabolisme ou d'un état inflammatoire chez un hôte selon la revendication 11 ou 12, dans laquelle ou lequel ledit composé est formulé pour une administration topique.

15. Utilisation selon la revendication 10, 12, 13 ou 14, ou composé destiné à être utilisé dans la modulation d'un trouble du métabolisme ou d'un état inflammatoire chez un hôte selon la revendication 11,12, 13 ou 14, dans laquelle ou lequel ladite modulation empêche un état médié par PPARγ.

16. Utilisation selon la revendication 10, 12, 13 ou 14, ou composé destiné à être utilisé dans la modulation d'un trouble du métabolisme ou d'un état inflammatoire chez un hôte selon la revendication 11,12, 13 ou 14, dans laquelle ou lequel ledit trouble ou ledit état est sélectionné dans le groupe constitué d'un DNID, d'une obésité et d'hypercholestérolémie, et par d'autres maladies et d'autres états inflammatoires médiés par l'isodisomie uniparentale.

17. Utilisation selon la revendication 10, 12, 13 ou 14, ou composé destiné à être utilisé dans la modulation d'un trouble du métabolisme ou d'un état inflammatoire chez un hôte selon la revendication 11,12, 13 ou 14, dans laquelle ou lequel ledit composé est formulé pour une administration parentérale.

18. Utilisation selon la revendication 10,12, 12, 13 ou 14, ou composé destiné à être utilisé dans la modulation d'un trouble du métabolisme ou d'un état inflammatoire chez un hôte selon la revendication 11,12, 13 ou 14, dans laquelle ou lequel ledit trouble du métabolisme est médié par PPARγ.

19. Utilisation selon la revendication 10,12, 12, 13 ou 14, ou composé destiné à être utilisé dans la modulation d'un trouble du métabolisme ou d'un état inflammatoire chez un hôte selon la revendication 11,12, 13 ou 14, dans laquelle ou lequel ledit trouble du métabolisme est un DNID.

20. Utilisation selon la revendication 10,12, 12, 13 ou 14, ou composé destiné à être utilisé dans la modulation d'un trouble du métabolisme ou d'un état inflammatoire chez un hôte selon la revendication 11, 12, 13 ou 14, dans laquelle ou lequel ledit état inflammatoire est une polyarthrite rhumatoïde ou une athérosclérose.

21. Utilisation selon les revendications 10 ou 12 à 20, ou composé destiné à être utilisé dans la modulation d'un trouble du métabolisme ou d'un état inflammatoire chez un hôte selon les revendications 11 ou 12 à 20, dans laquelle l'hôte est un humain.
